# EUROPEAN PATENT APPLICATION

(11) **EP 3 622 952 A1**
(43) Date of publication of application: **18.03.2020**
(21) Application number: 18798100.6
(22) Date of filing: 08.05.2018
(51) Int. Cl.: A61K 31/216, A23L 33/105, A61P 25/28, A61P 39/02

(54) **AGENT FOR PROMOTING DECOMPOSITION AND EXCRETION OF AMYLOID-**

(30) Priority: 12.05.2017 JP 2017095635; 06.04.2018 JP 2018073638
(71) Applicant: Kao Corporation, Chuo-ku Tokyo 103-8210 (JP)
(72) Inventor: YAMAMOTO, Masaki, Tochigi 321-3497 (JP); MISAWA, Koichi, Tokyo 1318501 (JP); ISHIDA, Keiko, Tochigi 321-3497 (JP); NISHIMURA, Hitomi, Tochigi 321-3497 (JP)
(74) Representative: Vossius & Partner Patentanwälte Rechtsanwälte mbB
(86) International application number: PCT/JP2018/017829
(87) International publication number: WO 2018/207790

(57) **Abstract**

Provided is an agent for promoting degradation and clearance of amyloid-β, the agent allowing continuous ingestion with high safety. The agent for promoting degradation and clearance of amyloid-β comprises a chlorogenic acid or a salt thereof as an active ingredient.

## Description

### [Field of the Invention]

The present invention relates to an agent for promoting degradation and clearance of amyloid-β that promotes degradation and clearance of amyloid-β.

### [Background of the Invention]

Alzheimer's disease is a neurodegenerative disease showing impairment of cognitive functions such as memory, learning, understanding, and judgement as main symptoms. It has been reported that dementia, of which Alzheimer's disease accounts for about 70%, is the third largest cause following cerebrovascular disease and fractures or falls, among the main causes leading to the need for nursing care or support for the elderly (2013 Comprehensive Survey of Living Conditions, Ministry of Health, Labour and Welfare). In addition, since the number of the patients is predicted to increase rapidly worldwide as the population aging, urgent action is required. However, at present, there is no effective preventive or therapeutic method for Alzheimer's disease, and development of new compound and preventive or therapeutic method are required.

The pathological characteristics of Alzheimer's disease are senile plaques and neurofibrillary tangles. Since it has been revealed that senile plaques are amyloid-β protein that has aggregated and fibrillated and extracellularly accumulated, the amyloid hypothesis has attracted attention as a mechanism that is the core of the onset and pathological progression. Amyloid precursor protein (APP) is cleaved stepwise by an enzyme reaction to produce amyloid-β, which is extracellularly insolubilized, aggregated, and accumulated to cause impairment in neurological function, such as neurotransmission, resulting in progression of symptoms and leading to onset of Alzheimer's disease (Non-Patent Literature 1).

Although the details of the role of amyloid-β have not been clarified, it is known that the generation of amyloid-β is increased with aging even in healthy subjects. Amyloid-β is generated in the brain and peripheral tissue, and the concentration in the brain is kept low by the homeostatic mechanism, in particular, by the clearance mechanism from the brain to the periphery. There are many routes for clearance of amyloid-β, and as a typical route, an efflux mechanism through a transporter present in cerebral blood vessels, such as low-density lipoprotein receptor-related protein (LRP1), is known. That is, it is important to maintain the homeostasis to appropriately regulate the production and clearance of amyloid-β in the brain for maintaining healthy cerebral function, and it is inferred that failure of such a mechanism by any cause leads to the onset of Alzheimer's disease (Non-Patent Literature 2).

Most of conventional or currently developing methods of preventing and treating Alzheimer's disease that has started with the production of amyloid-β are based on the amyloid hypothesis, and substance search is being conducted mainly on inhibition of APP cleaving enzyme, neutralization of amyloid-β, inhibition of aggregation, and promotion of degradation, of amyloid-β, and the like. For example, it has been reported that flavonoids, such as quercetin and kaempferol, which are natural product-derived components, have amyloid-β aggregation inhibitory activity (Patent Literature 1) and that a compound isolated from the extract of a plant of the genus Uncaria has a degrading action on aggregated amyloid-β (Patent Literature 2).

However, it is difficult to remove already accumulated amyloid-β by only the suppressive action on the production and aggregation of amyloid-β, and even if amyloid-β is degraded, it is difficult to obtain the treatment effect if the degraded amyloid-β cannot be excreted. In addition, the antibody therapy for amyloid-β causes, for example, side effects, and no clear efficacy against Alzheimer's disease has been recognized in such a method.

On the other hand, chlorogenic acids are found in plants such as coffee beans and potatoes, and an antioxidative action, a hypotensive action, etc. have been reported (Patent Literature 3). It has been also reported that di-O-caffeoylquinic acid in which two caffeic acids are linked by an ester bond has an action of protecting neuronal cells (Patent Literature 4).

However, it is not known that chlorogenic acids have an action of promoting degradation and clearance of amyloid-β.

Patent Literature 1: JP-A-2007-145839
Patent Literature 2: JP-A-2004-514679
Patent Literature 3: JP-A-2002-53464
Patent Literature 4: WO 2007/091613

Non-Patent Literature 1: Hardy JA, et al., Science, 1992, 256: 184
Non-Patent Literature 2: Zlokovic BV, et al., Nature Review, 2011, 12: 723

### [Summary of the Invention]

The present invention relates to the following aspects 1) to 14):
1) An agent for promoting degradation and clearance of amyloid-β, comprising a chlorogenic acid or a salt thereof as an active ingredient;
2) An agent for preventing, treating, or ameliorating Alzheimer's disease, comprising a chlorogenic acid or a salt thereof as an active ingredient;
3) A food for promoting degradation and clearance of amyloid-β, comprising a chlorogenic acid or a salt thereof as an active ingredient;
4) A food for preventing or ameliorating Alzheimer's disease, comprising a chlorogenic acid or a salt thereof as an active ingredient;
5) Use of a chlorogenic acid or a salt thereof for producing an agent for promoting degradation and clearance of amyloid-β;
6) Use of a chlorogenic acid or a salt thereof for producing an agent for preventing, treating, or ameliorating Alzheimer's disease;
7) Use of a chlorogenic acid or a salt thereof for producing a food for promoting degradation and clearance of amyloid-β;
8) Use of a chlorogenic acid or a salt thereof for producing a food for preventing or ameliorating Alzheimer's disease;
9) A chlorogenic acid or a salt thereof for use in promoting degradation and clearance of amyloid-β;
10) A chlorogenic acid or a salt thereof for use in prevention, treatment, or amelioration of Alzheimer's disease;
11) Non-therapeutic use of a chlorogenic acid or a salt thereof for promoting degradation and clearance of amyloid-β;
12) Non-therapeutic use of a chlorogenic acid or a salt thereof for preventing or ameliorating Alzheimer's disease;
13) A method of promoting degradation and clearance of amyloid-β, comprising administering to or ingesting in a subject in need thereof, an effective amount of a chlorogenic acid or a salt thereof; and
14) A method of preventing, treating, or ameliorating Alzheimer's disease, comprising administering to or ingesting in a subject in need thereof, an effective amount of a chlorogenic acid or a salt thereof.

### [Brief Description of Drawings]

[Figure 1] Figure 1 is a graph showing an activity of degrading aggregated amyloid by a chlorogenic acid.
[Figure 2] Figure 2 includes graphs showing an action of increasing amyloid-β efflux transporters (LRP1 and p-gp) by continuous ingestion of a chlorogenic acid.
[Figure 3] Figure 3 is a diagram showing an action of suppressing accumulation of amyloid-β in the brain by continuous ingestion of a chlorogenic acid (observation images of amyloid-β).
[Figure 4] Figure 4 is a graph showing an action of suppressing accumulation of amyloid-β in the hippocampus by continuous ingestion of a chlorogenic acid (accumulation rates of amyloid-β).
[Figure 5] Figure 5 is a graph showing an action of ameliorating the memory and learning ability by continuous ingestion of a chlorogenic acid (novel object recognition test).
[Figure 6] Figure 6 is a graph showing an action of ameliorating the memory and learning ability by continuous ingestion of a chlorogenic acid (Morris water maze test).

### [Detailed Description of the Invention]

The present invention provides an agent for promoting degradation and clearance of amyloid-β, the agent allowing continuous ingestion with high safety.

The present inventors variously examined highly safe ingredients that can be taken or ingested on a long-term basis and, as a result, found that a chlorogenic acid has excellent actions of promoting both degradation and clearance of amyloid-β and is also effective not only for prevention but also for amelioration of the Alzheimer's disease state in an Alzheimer's disease model animal.

According to the present invention, a drug and a food that promote degradation and clearance of amyloid-β and are useful for prevention, treatment, or amelioration of Alzheimer's disease are provided.

The term "chlorogenic acid" in the present invention is a generic name of a monocaffeoylquinic acid including 3-caffeoylquinic acid (3-CQA), 4-caffeoylquinic acid (4-CQA), and 5-caffeoylquinic acid (5-CQA) and a monoferuloylquinic acid including 3-feruloylquinic acid (3-FQA), 4-feruloylquinic acid (4-FQA), and 5-feruloylquinic acid (5-FQA). The content of the chlorogenic acid is defined based on the total amount of the above-mentioned six acids.

The "chlorogenic acid" of the present invention may include at least one of the above-mentioned six types of chlorogenic acids, and preferably includes a monocaffeoylquinic acid, and more preferably includes at least 5-caffeoylquinic acid, from the viewpoint of the action of promoting degradation and clearance of amyloid-β. The content of 5-caffeoylquinic acid is preferably 10 mass% or more, more preferably 20 mass% or more, more preferably 30 mass% or more, and further preferably 40 mass% or more, based on the total amount of the chlorogenic acids.

The chlorogenic acid may be in a salt form, and examples of the salt include a pharmaceutically acceptable salt, including a salt of an alkali metal such as sodium and potassium, a salt of an alkaline earth metal such as magnesium and calcium, a salt of an organic amine such as monoethanolamine, diethanolamine, and triethanolamine, and a salt of a basic amino acid such as arginine, lysine, histidine, and ornithine.

The chlorogenic acid or a salt thereof of the present invention can be, for example, a plant extract containing the chlorogenic acid or a salt thereof, a concentrate thereof, or a purified product thereof. Examples of the plant extract include those extracted from sunflower seeds, immature apples, coffee beans, Simon leaves, cones of pines, seed husks of pines, potatoes, sweet potatoes, sugar canes, wheat, nandin leaves, burdocks, carrots, cabbages, lettuces, artichokes, tomatoes, Mulukhiyah, eggplant peels, pears, plums, peaches, apricots, cherries, prunus mume fruits, coltsfoots, and grapes. In particular, from the viewpoint of, for example, the content of the chlorogenic acid, coffee bean extracts are preferred. Although the coffee beans may be deep-roasted coffee beans (L value: 16.8 or less), medium-roasted coffee beans (L value: higher than 16.8 and 24.2 or less), light-roasted coffee beans (L value: higher than 24.2 and 30.2 or less), slightly-roasted coffee beans (L value: higher than 30.2), or green coffee beans are more preferred, and green coffee beans are further preferred. Here, the "L value" is obtained by measuring the lightness of roasted coffee beans with a colorimeter, assuming that the L value of black as 0 and the L value of white as 100. The type of coffee tree may be any of Arabica, Robusta, Liberica, and Arabusta.

The coffee bean extract is preferably one from which caffeine has been removed, and the mass ratio of caffeine to chlorogenic acid, (caffeine/chlorogenic acid), is preferably 0.05 or less, more preferably 0.03 or less, further preferably 0.02 or less, further preferably 0.005 or less, further preferably 0.003 or less, and further preferably 0.001 or less from the viewpoint of flavor. The lower limit of the caffeine/chlorogenic acid ratio is not particularly limited, and the ratio may be 0.

In the coffee bean extract, the mass ratio of the sum of potassium (K) and sodium (Na) to chlorogenic acid, [(K + Na)/chlorogenic acid], is preferably 0.18 or less, more preferably 0.14 or less, further preferably 0.1 or less, further preferably 0.06 or less, from the viewpoint of flavor. The lower limit of the mass ratio, [(K + Na)/chlorogenic acid], is not particularly limited and may be 0. From the viewpoint of production efficiency, the lower limit is preferably 0.0001 and further preferably 0.001.

Preferred examples of the coffee bean extract to be used in the present invention include green coffee bean extracts in which the mass ratio of caffeine to chlorogenic acid, (caffeine/chlorogenic acid), is 0.02 or less and the content of 5-caffeoylquinic acid in the total amount of the chlorogenic acids, (5-CQA/total chlorogenic acids), is 40 mass% or more. More preferred examples include green coffee bean extracts in which the mass ratio, (caffeine/chlorogenic acid), is 0.001 or less and the content, (5-CQA/total chlorogenic acids), is 40 mass% or more.

The chlorogenic acid or a salt thereof of the present invention may be extracted, concentrated, and purified by any methods and conditions, and known methods and conditions can be adopted. Among them, extraction with an ascorbic acid aqueous solution, a citric acid aqueous solution, or hot water is preferred.

As a raw material of the chlorogenic acid or a salt thereof, a commercially available chlorogenic acid-containing preparation may be used, and examples thereof include Flavor Holder RC (T. Hasegawa co., Ltd.), Green Coffee Bean Extract-P (manufactured by Oryza Oil & Fat Chemical Co., Ltd.), Svetol (manufactured by Nurex Co., Ltd.), and OXCH 100 (manufactured by Toyo Hakko Co., Ltd.).

As shown in examples below, the chlorogenic acid has an action of degrading aggregated amyloid-β, and also enhances expression of amyloid-β efflux transporter genes (LRP1 gene and p-gp gene) in the hippocampus to suppress accumulation of amyloid-β in the brain. Furthermore, it has been verified that the chlorogenic acid shows an effect of ameliorating the memory and learning ability in an Alzheimer's disease model animal.

Accordingly, the chlorogenic acid or a salt thereof can serve as an agent for promoting degradation and clearance of amyloid-β or an agent for preventing, treating, or ameliorating Alzheimer's disease. The chlorogenic acid or a salt thereof can be used for promoting degradation and clearance of amyloid-β and for preventing, treating, or ameliorating Alzheimer's disease and also can be used for producing an agent for promoting degradation and clearance of amyloid-β or an agent for preventing, treating, or ameliorating Alzheimer's disease.

Here, the "use" can be administration to or ingestion in a human or a non-human animal and may be therapeutic or non-therapeutic. The term "non-therapeutic" refers to a concept that does not include medical practice, i.e., a concept that does not include a method of surgery, treatment or diagnosis for a human, more specifically, a concept that does not include a method of performing surgery, treatment, or diagnosis for a human by a doctor or a person who has received instruction of a doctor.

In the present invention, the phrase "promotion of degradation and clearance of amyloid-β" means that degradation of aggregated amyloid-β protein accumulated in the brain is promoted and clearance of amyloid-β protein from the brain is promoted to suppress accumulation of amyloid-β in the brain.

The term "Alzheimer's disease" in the present invention refers to a dementing disorder that exhibits symptoms such as cognitive impairment (memory disorder, orientation disturbance, learning impairment, attention disorder, and space cognitive function), personality changes caused by increase and accumulation of amyloid-β in the brain and social incompatibility.

In the present specification, the term "prevention" refers to prevention or delay of the onset of a disease or symptom in an individual or a decrease in the risk of the onset of a disease or symptom in an individual.

The term "amelioration" refers to a change for the better in a disease, symptom, or condition, prevention or delay of worsening of a disease, symptom, or condition, or reverse, prevention, or delay of progression of a disease or symptom.

The term "treatment" encompasses not only complete cure of a disease but also amelioration of a symptom.

The agent for promoting degradation and clearance of amyloid-β and the agent for preventing, ameliorating, or treating Alzheimer's disease of the present invention may be per se a drug, quasi-drug, supplement, or food for promoting degradation and clearance of amyloid-β or preventing, ameliorating, or treating Alzheimer's disease or may be a material or preparation to be used for blending in the drug, quasi-drug, or food.

The term "food" encompasses foods, functional foods, foods for patients, foods for specified health uses, and supplements which are based on concepts of promotion of degradation and clearance of amyloid-β and prevention or amelioration of Alzheimer's disease, and which indicate the concepts as necessary. These foods have been approved to indicate the function and can be distinguished from common foods.

Examples of the dosage form of the drug (including quasi-drug) include oral administration in the form of, for example, tablets, capsules, granules, powders, and syrups and parenteral administration in the form of, for example, injection, suppository, and inhalation. Preparations of such various dosage forms can be prepared from the chlorogenic acid or a salt thereof of the present invention alone or in appropriate combination with, for example, a pharmaceutically acceptable excipient, binder, filler, disintegrator, surfactant, lubricant, dispersant, buffer, preservative, corrective agent, flavor, coating, carrier, diluent, and medicinal ingredient other than the chlorogenic acid or a salt thereof of the present invention. Among these dosage forms, preferred is oral administration.

Examples of the form of the food include foods and drinks, such as soft drinks, tea drinks, coffee drinks, fruit juice drinks, carbonated drinks, jellies, wafers, biscuits, bread, noodles, and sausages; various foods, such as nutritional foods; and a nutritional composition in the same forms as those of the above-described oral administration (tablets, capsules, syrups, etc.).

Foods of such various forms can be prepared from the chlorogenic acid or a salt thereof of the present invention alone or in appropriate combination with, for example, other food ingredient, a solvent, a softener, an oil, an emulsifier, an antiseptic, an acidifier, a sweetener, a bittering agent, a flavor, a stabilizer, a colorant, an antioxidant, a moisturizer, a thickener, and an active ingredient other than the chlorogenic acid or a salt thereof of the present invention.

Although the content of the chlorogenic acid or a salt thereof of the present invention in the drugs (including quasi-drugs) and foods varies depending on the form of use, usually, the content is, in terms of a chlorogenic acid, preferably 0.001 mass% or more, more preferably 0.01 mass% or more, and further preferably 0.1 mass% or more and preferably 90 mass% or less, more preferably 60 mass% or less, and further preferably 30 mass% or less based on the total amount. In addition, the content is preferably from 0.001 to 90 mass%, more preferably from 0.01 to 60 mass%, and further preferably from 0.1 to 30 mass%.

Although the amount of the drug (including quasi-drug) and food to be administered or ingested can be appropriately determined, usually, the amount is preferably 100 mg or more, in terms of a chlorogenic acid, more preferably 300 mg or more and preferably 3000 mg or less, more preferably 1000 mg or less per day for an adult (60 kg). In the present invention, such an amount is preferably administered or ingested at one time.

The preparation is administered or ingested according to an arbitrary schedule. Although the administration or ingestion period is not particularly limited, repeated and continuous administration or ingestion is preferred, and the continuous administration or ingestion is performed more preferably for 7 or more days and further preferably for 28 or more days.

Although the subject of administration or ingestion may be any human or non-human animal that needs or wishes prevention or amelioration of a reduction in cognitive function, administration to or ingestion in a human is effective. Here, the reduction in cognitive function is a symptom that is observed in cognitive functional disorder typically represented by Alzheimer's disease and refers to, for example, memory and learning impairment (impairment in ability of acquiring new information and keeping it in memory), impairment in inference and handling of complicated work, poor judgment, impairment in visuospatial ability, speech disorder, and a condition of interfering with work or daily activities or causing a decrease in executive function accompanied with changes in personality, action, or behavior. In addition, the human who needs or wishes such prevention of a reduction in cognitive function refers to, for example, a healthy subject scoring 27 to 30 points in a Mini-Mental State Examination (MMSE), which is a most widely and internationally used cognitive function test, and the human who needs or wishes amelioration of a reduction in cognitive function encompasses a human who is suspected of mild cognitive impairment scoring 22 to 26 points in the above-mentioned test and a human who has a high risk of cognitive impairment, such as dementia, scoring 21 or less points.

Regarding the above-described embodiment, the present invention discloses the following aspects:
<1> An agent for promoting degradation and clearance of amyloid-β, comprising a chlorogenic acid or a salt thereof, or a plant extract comprising the same, as an active ingredient;
<2> An agent for preventing, treating, or ameliorating Alzheimer's disease, comprising a chlorogenic acid or a salt thereof, or a plant extract comprising the same, as an active ingredient;
<3> A food for promoting degradation and clearance of amyloid-β, comprising a chlorogenic acid or a salt thereof, or a plant extract comprising the same, as an active ingredient;
<4> A food for preventing or ameliorating Alzheimer's disease, comprising a chlorogenic acid or a salt thereof, or a plant extract comprising the same, as an active ingredient;
<5> Use of a chlorogenic acid or a salt thereof, or a plant extract comprising the same, for producing an agent for promoting degradation and clearance of amyloid-β;
<6> Use of a chlorogenic acid or a salt thereof, or a plant extract comprising the same, for producing an agent for preventing, treating, or ameliorating Alzheimer's disease;
<7> Use of a chlorogenic acid or a salt thereof, or a plant extract comprising the same, for producing a food for promoting degradation and clearance of amyloid-β;
<8> Use of a chlorogenic acid or a salt thereof, or a plant extract comprising the same, for producing a food for preventing or ameliorating Alzheimer's disease;
<9> The use according to aspect <5> or <6>, wherein the agent is in an oral administration form;
<10> A chlorogenic acid or a salt thereof, or a plant extract comprising the same, for use in promoting degradation and clearance of amyloid-β;
<11> A chlorogenic acid or a salt thereof, or a plant extract comprising the same, for use in preventing, treating, or ameliorating Alzheimer's disease;
<12> The chlorogenic acid or a salt thereof, or a plant extract comprising the same according to aspect <10> or <11>, wherein the chlorogenic acid or a salt thereof, or a plant extract comprising the same is orally administered;
<13> Non-therapeutic use of a chlorogenic acid or a salt thereof or a plant extract comprising the same, for promoting degradation and clearance of amyloid-β;
<14> Non-therapeutic use of a chlorogenic acid or a salt thereof or a plant extract comprising the same, for preventing or ameliorating Alzheimer's disease;
<15> The non-therapeutic use according to aspect <13> or <14>, wherein the chlorogenic acid or a salt thereof, or a plant extract comprising the same is orally administered;
<16> A method of promoting degradation and clearance of amyloid-β, comprising administering to or ingesting in a subject in need thereof, an effective amount of a chlorogenic acid or a salt thereof, or a plant extract comprising the same;
<17> A method of preventing, treating, or ameliorating Alzheimer's disease, comprising administering to or ingesting in a subject in need thereof, an effective amount of a chlorogenic acid or a salt thereof, or a plant extract comprising the same;
<18> The method according to aspect <16> or <17>, wherein the administering is oral administration;
<19> In aspects <1> to <18>, the chlorogenic acid comprises at least 5-caffeoylquinic acid;
<20> In aspects <1> to <19>, the plant extract comprising a chlorogenic acid or a salt thereof is a coffee bean extract, preferably a green coffee bean extract;
<21> In aspect <20>, the green coffee bean extract has preferably a mass ratio of caffeine to the chlorogenic acid, (caffeine/chlorogenic acid), being 0.02 or less and a content of 5-caffeoylquinic acid in the total amount of the chlorogenic acids, (5-CQA/total chlorogenic acids), being 40 mass% or more, and more preferably the ratio (caffeine/chlorogenic acid) being 0.001 or less and the content (5-CQA/total chlorogenic acids) being 40 mass% or more;
<22> In the agent for promoting degradation and clearance of amyloid-β according to aspects <1> and <5>, the agent for preventing, treating, or ameliorating Alzheimer's disease according to aspects <2> and <6>, the food for promoting degradation and clearance of amyloid-β according to aspects <3> and <7>, and the food for preventing or ameliorating Alzheimer's disease according to aspects <4> and <8>, the content of the active ingredient is, in terms of a chlorogenic acid, preferably 0.001 mass% or more, more preferably 0.01 mass% or more, and further preferably 0.1 mass% or more; and preferably 90 mass% or less, more preferably 60 mass% or less, and further preferably 30 mass% or less based on the total amount; and in addition, the content is preferably from 0.001 to 90 mass%, more preferably from 0.01 to 60 mass%, and further preferably from 0.1 to 30 mass%; and
<23> In aspects <10> to <18>, the dose per day for an adult is preferably 100 mg or more, more preferably 300 mg or more, and preferably 3000 mg or less, more preferably 1000 mg or less, in terms of a chlorogenic acid.

### [Examples]

Production Example 1: Preparation of chlorogenic acid-containing composition
1) Green Robusta coffee beans were subjected to extraction with hot water, and the resulting liquid extract was dried by spray drying to obtain a crude chlorogenic acid-containing composition. The crude chlorogenic acid-containing composition contained 32.3 mass% of the chlorogenic acid and 9.8 mass% of caffeine, where the mass ratio (caffeine/chlorogenic acid) was 0.303, and the mass ratio ((K + Na)/chlorogenic acid) was 0.24.
2) The crude chlorogenic acid-containing composition (189 g) was mixed with an ethanol aqueous solution (756 g) having an ethanol concentration of 52.4 mass%, acid clay (94.5 g, Mizuka-Ace #600, manufactured by Mizusawa Industrial Chemicals, Ltd.), and a filter aid (10.7 g, Solka Floc, manufactured by Nippon Mining Procurement, Inc.) to prepare a chlorogenic acid-containing slurry (1,051 g). The chlorogenic acid-containing slurry had a pH of 5.7.
3) The chlorogenic acid-containing slurry (1,051 g) was filtered through filter paper No. 2 on which diatomite was deposited as a precoating agent, and an ethanol aqueous solution (189 g) having an ethanol concentration of 52.4 mass% was then passed through the filter paper to collect the filtrate (1,054 g).
4) Subsequently, a column filled with activated carbon (132 mL, SHIROSAGI WH2C, manufactured by Japan EnviroChemicals, Ltd.) and a column filled with an H-type cation exchange resin (105 mL, SK1BH, manufactured by Mitsubishi Chemical Corp.) were connected to each other, and the filtrate (1,019 g) and then an ethanol aqueous solution (231 g) having an ethanol concentration of 52.4 mass% were passed through the columns to collect 1,072 g of the eluted column-treated liquid. The amount of the activated carbon used was 0.81 mass (g/g) times the amount of the chlorogenic acid in the filtrate. The amount of the ion exchange resin used was 0.74 (mL/g) based on the solid content in the crude chlorogenic acid-containing composition.
5) The column-treated liquid (1,038 g) was filtered through a 0.2 µm membrane filter, and subsequently ethanol was distilled away by a rotary evaporator to obtain 225 g of a chlorogenic acid-containing solution. The chlorogenic acid-containing solution contained 22.6 mass% of the chlorogenic acid and 0.29 mass% of caffeine, where the mass ratio (caffeine/chlorogenic acid) was 0.013, the ethanol concentration was 0 mass%, and the pH was 3.1.
6) The chlorogenic acid-containing solution was diluted with distilled water to adjust the concentration of the chlorogenic acid to 3 mass%. The resulting diluted solution (10 g) was sampled in a centrifuge tube and was then centrifuged at 3000 rpm at 15°C for 60 minutes. The supernatant was lyophilized to obtain chlorogenic acid-containing purified coffee polyphenol (CPP). In the CPP, the content of 5-CQA was 45.6% based on the total amount of the chlorogenic acids. The composition ratios of the chlorogenic acid in the CPP are shown in Table 1.

**[Table 1]**

| (%) | | | | | |
|---|---|---|---|---|---|
| 3-CQA | 5-CQA | 4-CQA | 3-FQA | 5-FQA | 4-FQA |
| 7.0 | 17.8 | 7.5 | 1.3 | 3.9 | 1.5 |

| | | | | | |
|---|---|---|---|---|---|
| CQA: caffeoylquinic acid, FQA: feruloylquinic acid | | | | | |

### Example 1: Action of degrading aggregated amyloid-P by chlorogenic acid

The direct activity of degrading aggregated amyloid by chlorogenic acid (5-CQA) was examined using a thioflavin T (ThT) fluorescence assay.

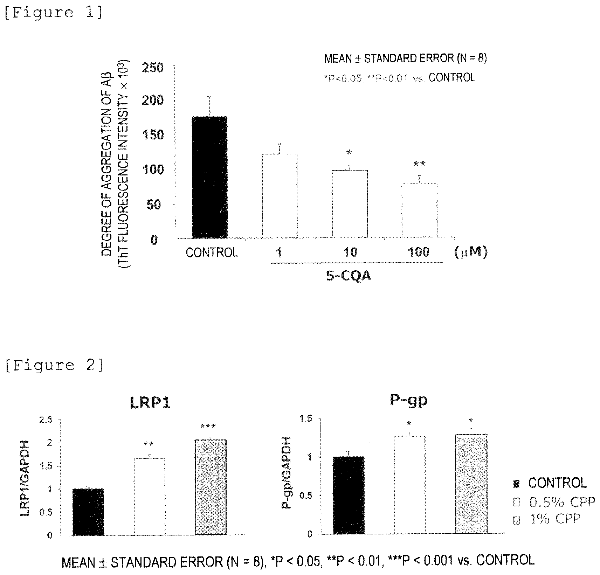

An amyloid-β peptide (Human, 1-42, Peptide Institute, Inc.) was dissolved in dimethyl sulfoxide (DMSO) to 2.5 mM and was further adjusted to 250 µM with sterile water, followed by incubation at 37°C for 7 days to promote the aggregation reaction. After the reaction, chlorogenic acid (5-CQA, Cayman Chemical Company) was added to the aggregated amyloid-β (10 mass%) at a final concentration of 1, 10, or 100 µM, followed by further incubation at 37°C for 7 days. As a control, 0.5% DMSO, which was used as the solvent of the chlorogenic acid, was added. After the reaction, ThT (Sigma-Aldrich, final concentration: 5 µM) dissolved in 50 mM glycine buffer (pH 8.5) was added to the reaction solution in a 96-well plate (ThermoFisher Scientific), followed by a reaction at room temperature for 15 minutes while gently stirring under light shielding. After the reaction, fluorescence was detected at 450 nm (excitation) and 480 nm (emission) using a fluorescence plate reader (Ensight 3400, PerkinElmer Co., Ltd.) to measure the degree of aggregation of amyloid-β. Figure 1 demonstrates that the chlorogenic acid has an activity of degrading aggregated amyloid in a dose-dependent manner.

### Example 2: Action of increasing amyloid-β efflux transporter by continuous ingestion of chlorogenic acid

Using 12 weeks old male C57BL/6 mice, diet in which 0.5 mass% or 1 mass% of the chlorogenic acid-containing purified coffee polyphenol (CPP) prepared in Production Example 1 was added to standard diet was ingested for 4 weeks in an experimental group, and the standard diet only was ingested in a control group (n = 8 in each group). The standard diet was composed of 10 mass% of corn oil, 20 mass% of casein, 4 mass% of cellulose, 3.5 mass% of mineral mixture, 1 mass% of vitamin mixture, and 61.5 mass% of potato starch, and the CPP-containing diet was produced by using CPP in replacement of an equivalent amount of the potato starch. After the ingestion period, hippocampus was isolated under deep anesthesia, and total RNA was extracted from the isolated brain tissue using a RNeasy universal mini kit (QIAGEN). Complementary DNA was synthesized from the total RNA by a reverse transcription reaction (37°C, 60 minutes) using a High Capacity RNA-to-cDNA kit (ThermoFisher Scientific). The synthesized cDNA (100 ng) was mixed with TaqMan (registered trademark) Fast Universal PCR Master Mix (ThermoFisher Scientific) and purchased primers and probes designed in advance using TaqMan (registered trademark) Gene Expression Assay (ThermoFisher Scientific), and the gene expression levels of low-density lipoprotein receptor-related protein 1 (LRP1) and p-glycoprotein (p-gp), which are efflux transporters of amyloid-β, were analyzed by a real-time PCR method. The expression levels of the examined two amyloid-β efflux transporter genes were both significantly increased in the hippocampus by ingestion of CPP (experimental group) compared to the control group (Figure 2).

### Example 3: Action of suppressing accumulation of amyloid-β in the brain by continuous ingestion of chlorogenic acid

APP/PS2 double-transgenic mice (APP/PS2WTg), which are Alzheimer's disease model mice highly expressing amyloid-β, were produced using APP-Tg mice (Taconic Biosciences, Inc.) on a B6;SJL background carrying K670N and M671L mutations (Swedish mutation) of human amyloid precursor protein (APP) in the whole body and PS2-Tg mice (Oriental Yeast Co., Ltd.) on a C57BL/6J mice background carrying N141 mutation of human presenilin 2 (PS2) in the whole body. Spermatozoa collected from male APP-Tg mice (10 or more weeks old) and oocytes of female PS2-Tg mice (4 or more weeks old) were subjected to in vitro fertilization to produce APP/PS2WTg mice. Using 5 weeks old male APP/PS2WTg mice in the test, diet in which 1 mass% of the chlorogenic acid-containing purified coffee polyphenol (CPP) prepared in Production Example 1 was added to the same standard diet as in Example 2 was ingested for 20 weeks in an experimental group, and the standard diet only was ingested in a control group (n = 6 to 8 in each group). After the ingestion period, perfusion was performed with ice-cold saline (20 mL) and 4% paraformaldehyde phosphate buffer (PFA, 20 mL) under deep anesthesia, and the whole brain was then isolated and immersed in 4% PFA at 4°C for fixation. Subsequently, a paraffin block was prepared, and paraffin sections of 4 µm thickness were prepared for the dorsal hippocampus region. The brain sections were deparaffinized and immersed in 90% formic acid at room temperature for 5 minutes then in a methanol solution containing 0.1% aqueous hydrogen peroxide at room temperature for 30 minutes to inactivate endogenous peroxidase. After washing with PBS/Tween 20 (PBS-T), a primary antibody reaction using an anti-human amyloid-β antibody (1:200, Immuno-Biological Laboratories Co., Ltd.) was performed at room temperature for 60 minutes. After the reaction and then washing with PBS-, HRP-labeled streptavidin was dropwise added thereto, a reaction was performed at room temperature for 5 minutes, and a coloring reaction was then performed with a DAB kit (DAKO). After nuclear staining with hematoxylin, washing with running water, dehydration, clearing, and inclusion were performed to produce slide samples. Observation was performed with a fluorescence microscope (BZ-X710, Keyence Corporation), and the staining-containing area (amyloid accumulation ratio) was calculated using the attached analysis software (BZ-II application). Four sections were produced for one individual, and the average staining-containing area value of four sections was defined as the value of the individual.

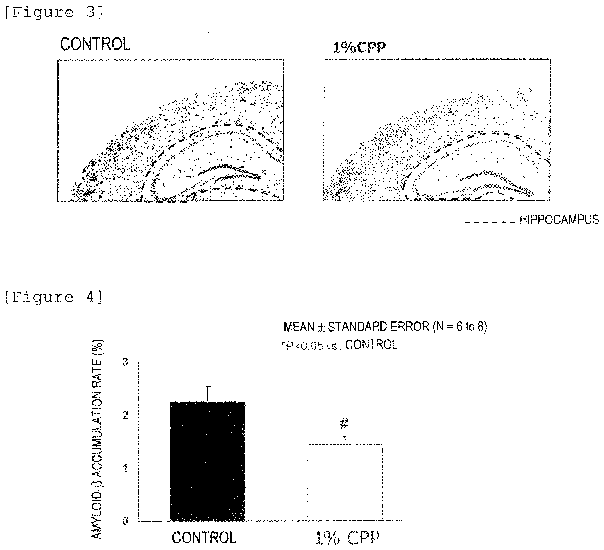

It was clear from the observation image (Figure 3) that amyloid-β accumulation in the brain was obviously decreased in the CPP ingestion group (experimental group) compared to the control group. In addition, it was recognized from the calculation of the amyloid accumulation ratio that accumulation in the hippocampus, which plays an important role particularly in memory, was significantly decreased (Figure 4).

### Example 4: Action of ameliorating memory and learning ability by continuous ingestion of chlorogenic acid

Using 5 weeks old male APP/PS2WTg as in Example 3 and the same weeks old background animal C57BL/6 thereof (healthy control group), diet in which 1 mass% of the chlorogenic acid-containing purified coffee polyphenol (CPP) prepared in Production Example 1 was added to standard diet was ingested in an experimental group, and the standard diet only was ingested in a control group and a healthy control group (n = 12 to 15 in each group). A novel object recognition test and a Morris water maze test were implemented from the 18th week of the start of the test food ingestion to measure influence on the memory and learning ability. In the novel object recognition test for evaluating the visual cognitive function (long-term memory), each mouse was placed in a plastic cage (Width: 220, Depth: 320, Height: 130 mm) and was acclimated to the environment for 10 minutes (acclimation trial). On the next day, the mouse was placed in the cage installed with objects (blocks: A1 and A2), and the behavior of the mouse was video-recorded for 10 minutes (training trial). Two hours after the training trial, only one object was changed to a new object (golf ball: B), the mouse was placed in the cage again, and the behavior of the mouse was video-recorded for 5 minutes (retention trial).

Using the recorded moving images, the number of contacts of the mouse with each object during the training trial and the retention trial was counted as the number of searches using a counter. The rate (%) of the number of searches for each object to the total number of searches for the objects was calculated.

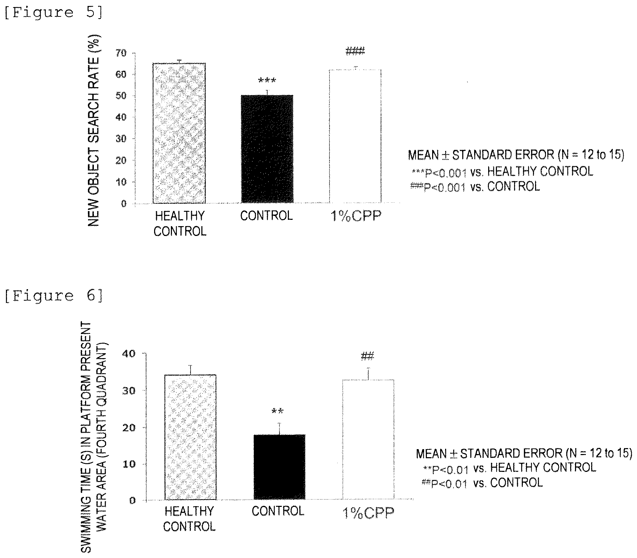

The search rate for the new object during the retention trial in the Alzheimer's disease model APP/PS2WTg control group was significantly low compared to the healthy control group, and a reduction in memory and learning ability (visual cognitive function) was observed. In contrast, in the experimental group that ingested 1 mass% of CPP, the memory and learning ability (visual cognitive function) was significantly improved compared to the control group, and it was revealed that the memory and learning ability (visual cognitive function) was retained at almost the same level as that in the healthy control group (Figure 5).

Subsequently, in the Morris water maze test for evaluating the space cognitive function of long-term memory, a visually indistinguishable transparent platform (diameter: 12 cm) was installed in a circular pool with a diameter of 148 cm, and the pool was filled with water (water temperature: 18°C) such that the platform was hidden by the water and was used. The platform was installed at the center of one of the four quadrants of the pool (hereinafter, referred to as the fourth quadrant), and a bulb was installed on the periphery of the pool as a spatial cue. A mouse was placed in the pool in such a manner that the head of the mouse was directed toward the wall of the pool and was allowed to swim to reach the platform for up to 90 seconds. When the mouse did not reach the platform, the mouse was placed on the platform for 30 seconds and then returned to the breeding cage. This procedure was carried out 3 times a day for 4 days (12 times in total) (acquisition trial). On the 5th day, the mouse was placed in the pool from which the platform was removed in such a manner that the head of the mouse was directed to the wall of the pool, and the time (fourth quadrant swimming time) for which the mouse stayed in the fourth quadrant (the quadrant in which the platform was installed during acquisition trial) within 90 seconds was video-recorded (probe trial).

From the recorded moving image, the fourth quadrant swimming time during the probe trial was calculated using analysis software (EthoVision XT: Noldus Information Technology).

The fourth quadrant swimming time during the probe trial in the Alzheimer's disease model APP/PS2WTg control group was significantly short compared to the healthy control group, and a reduction in memory and learning ability (space cognitive function of long-term memory) was observed. In contrast, in the experimental group that ingested 1 mass% of CPP, the memory and learning ability (space cognitive function of long-term memory) was significantly improved compared to the control group, and it was revealed that the memory and learning ability (space cognitive function of long-term memory) was retained at almost the same level as that in the healthy control group (Figure 6).

## Claims

1. An agent for promoting degradation and clearance of amyloid-β, comprising a chlorogenic acid or a salt thereof as an active ingredient.

2. An agent for preventing, treating, or ameliorating Alzheimer's disease, comprising a chlorogenic acid or a salt thereof as an active ingredient.

3. A food for promoting degradation and clearance of amyloid-β, comprising a chlorogenic acid or a salt thereof as an active ingredient.

4. A food for preventing or ameliorating Alzheimer's disease, comprising a chlorogenic acid or a salt thereof as an active ingredient.

5. The agent for promoting degradation and clearance of amyloid-β according to claim 1, the agent for preventing, treating, or ameliorating Alzheimer's disease according to claim 2, the food for promoting degradation and clearance of amyloid-β according to claim 3, and the food for preventing or ameliorating Alzheimer's disease according to claim 4, wherein the chlorogenic acid comprises at least 5-caffeoylquinic acid.

6. Use of a chlorogenic acid or a salt thereof for producing an agent for promoting degradation and clearance of amyloid-β.

7. Use of a chlorogenic acid or a salt thereof for producing an agent for preventing, treating, or ameliorating Alzheimer's disease.

8. Use of a chlorogenic acid or a salt thereof for producing a food for promoting degradation and clearance of amyloid-β.

9. Use of a chlorogenic acid or a salt thereof for producing a food for preventing or ameliorating Alzheimer's disease.

10. The use according to claim 6 or 7, wherein the agent is in an oral administration form.

11. A chlorogenic acid or a salt thereof for use in promoting degradation and clearance of amyloid-β.

12. A chlorogenic acid or a salt thereof for use in preventing, treating, or ameliorating a disease.

13. The chlorogenic acid or a salt thereof according to claim 11 or 12, wherein the chlorogenic acid or a salt thereof is orally administered.

14. Non-therapeutic use of a chlorogenic acid or a salt thereof for promoting degradation and clearance of amyloid-β.

15. Non-therapeutic use of a chlorogenic acid or a salt thereof for preventing or ameliorating Alzheimer's disease.

16. The non-therapeutic use according to claim 14 or 15, wherein the chlorogenic acid or a salt thereof is orally administered.

17. A method of promoting degradation and clearance of amyloid-β, comprising administrating to or ingesting in a subject in need thereof, an effective amount of a chlorogenic acid or a salt thereof.

18. A method of preventing, treating, or ameliorating Alzheimer's disease, comprising administering to or ingesting in a subject in need thereof, an effective amount of a chlorogenic acid or a salt thereof.

19. The method according to claim 17 or 18, wherein the administering is oral administration.
